Europäisches Patentamt

European Patent Office

Office européen des brevets

(19)

(11) Veröffentlichungsnummer : **0 010 236**
**B2**

(12)

# NEUE EUROPÄISCHE PATENTSCHRIFT

(45) Veröffentlichungstag der neuen Patentschrift :
**15.04.87**

(51) Int. Cl.⁴ : **C 07 C 29/03**, C 07 C 31/20

(21) Anmeldenummer : **79103815.1**

(22) Anmeldetag : **05.10.79**

(54) Verfahren zur Herstellung von 2,3-Dimethyl-2,3-butandiol.

(30) Priorität : **13.10.78 DE 2844637**

(43) Veröffentlichungstag der Anmeldung :
**30.04.80 Patentblatt 80/09**

(45) Bekanntmachung des Hinweises auf die Patenterteilung : **09.02.83 Patentblatt 83/06**

(45) Bekanntmachung des Hinweises auf die Entscheidung
über den Einspruch : **15.04.87 Patentblatt 87/16**

(84) Benannte Vertragsstaaten :
**BE CH FR GB IT**

(56) Entgegenhaltungen :
FR-A- 1 047 126
GB-A-   449 060
GB-A-   634 118
US-A- 2 492 201
US-A- 3 173 968
BULLETIN DE LA SOCIETE CHIMIQUE DE FRANCE,
Vol. 11, November 1970, Paris, FR, A. LEBOUC:
"Hydroxylation d'alcools primaires gamma-éthyléniques trans. I- Préparation et étude stéréochimique
des triols obtenus", Seiten 4099-4104
D. Swern, Organic Peroxides, Vol. II, Wiley (1971), S.
400-401
D. Swern, J. Am. Chem. Soc, 67, 1786-1789 (1945)
F. Asinger und B. Fell, Erdöl und Kohle-Erdgas-Petrochemie, 19. Jahrgang, April 1966 Nr. 4, S. 263

(73) Patentinhaber : **Ruhrchemie Aktiengesellschaft**
**Bruchstrasse 219**
**D-4200 Oberhausen 13 (DE)**

(72) Erfinder : **Cornils, Boy, Dr. Dipl.-Chem.**
**Friedrich-Ebert-Strasse 45**
**D-4220 Dinslaken (DE)**
Erfinder : **Weber, Jürgen, Dr. Dipl.-Chem.**
**Bunsenstrasse 17**
**D-4200 Oberhausen 13 (DE)**
Erfinder : **Bernhagen, Wolfgang, Dr. Dipl.-Chem.**
**Witthausstrasse 19**
**4330 Mülheim (DE)**
Erfinder : **Springer, Helmut**
**Drostenkampstrasse 24**
**D-4200 Oberhausen 13 (DE)**

(74) Vertreter : **Reichelt, Karl-Heinz, Dr.**
**m. Br. Ruhrchemie Aktiengesellschaft Abt. PLD Post-**
**fach 13 01 60**
**D-4200 Oberhausen 11 (DE)**

EP 0 010 236 B2

## Beschreibung

Die vorliegende Erfindung betrifft ein Verfahren zur Herstellung von 2,3-Dimethyl-2,3-butandiol (Pinakol).

Es ist bekannt, Diole durch Hydroxylierung von Olefinen mit Wasserstoffperoxid in Gegenwart von Metalloxiden als Katalysatoren herzustellen. Nach einer anderen Variante dieser Reaktion setzt man die ungesättigten Verbindungen in Gegenwart von Säuren, bevorzugt Carbonsäuren, mit Wasserstoffperoxid um. Als hydroxylierendes Agens wirkt in diesem Falle die unter den Reaktionsbedingungen entstehende Percarbonsäure. Bei der Hydroxylierung werden zunächst Ester gebildet, die in einem zweiten Reaktionsschritt zum entsprechenden Diol verseift werden.

Die Hydroformylierung olefinischer Verbindungen ist literaturmäßig mehrfach belegt.

Wie in der FR-PS 1 047 126 beschrieben, lassen sich längerkettige Fettalkohole zu den entsprechenden Polyalkoholen umwandeln, indem man Fettalkohol, Wasserperoxid und Ameisensäure miteinander vermischt und reagieren läßt.

Die Umsetzung von kurzkettigen Olefinen, die eine polare Gruppe enthalten können, beschreibt die GB-PS 634 118.

Hierbei wird das umzusetzende Olefin in ein Gemisch, das aus Ameisensäure und Wasserstoffperoxid besteht, eingeleitet. Die Umsetzung erfolgt bei tiefen Temperaturen und erfordert Reaktionszeiten bis zu 12 Tagen. Die US-PS 2 492 201 befaßt sich mit der Umsetzung von Olefinen, insbesondere mit der Reaktion von Ölsäure zu 9.10-Dihydroxystearinsäure in Gegenwart von Perameisensäure und Mischungen aus Wasserstoffperoxid und Ameisensäure. Olefin und Ameisensäure werden vorgelegt und in die Mischung wird Wasserstoffperoxid eingeleitet.

Ein anderes Verfahren zur Umsetzung von ethylenisch ungesättigten Alkoholen ist in Bull. Soc. Chem. 11, Nov. 1970 angegeben. Man legt den ungesättigten Alkohol vor und gibt anschließend Ameisensäure und Wasserstoffperoxid zu.

Die vorstehend geschilderten Verfahren haben den Nachteil, daß sie stets mit der Bildung freier Persäuren in höherer Konzentration verbunden sind. Da Peroxide und Olefine zur Bildung explosiver Gemische führen, erweisen sich diese Prozesse aus Sicherheitsgründen für eine Anwendung im technischen Maßstab als ungeeignet. Darüber hinaus mindert die hohe Persäurekonzentration die Wertproduktausbeute, weil das primär erhaltene Glykol durch überschüssige Persäure zu unerwünschten Nebenprodukten weiterreagiert.

Es bestand daher die Aufgabe, ein Verfahren zu entwickeln, das die Herstellung von 2,3-Dimethyl-2,3-butandiol auf einfachem und gefahrlosem Wege ermöglicht und sicherstellt, daß das Diol in hohen Ausbeuten erhalten wird.

Erfindungsgemäß wird diese Aufgabe gelöst durch ein Verfahren zur Herstellung von 2,3-Dimethyl-2,3-butandiol durch Umsetzung von 2,3-Dimethylbuten mit Wasserstoffperoxid und Ameisensäure und Spaltung des gebildeten Formiates. Es ist dadurch gekennzeichnet, daß man gleichzeitig aber getrennt ein Gemisch aus 2,3-Dimethylbuten mit bis zu 30 Gew.-% 2,3-Dimethylbuten-1 und Wasserstoffperoxid bei 50 bis 70 °C unter Rühren in Ameisensäure einleitet.

Überraschenderweise hat sich herausgestellt, daß nicht nur reines 2,3-Dimethylbuten-2, sondern auch Gemische aus 2,3-Dimethylbuten-2 und 2,3-Dimethylbuten-1 mit einem Anteil bis zu etwa 30 Gew.-% (bezogen auf das Gesamtgemisch) 2,3-Dimethylbuten-1 zum Einsatz kommen können, ohne daß andere Reaktionsprodukte als das gewünschte 2,3-Dimethyl-2,3-butandiol gebildet werden. Dieses Verhalten des Olefingemisches war nicht zu erwarten, da 2,3-Dimethylbuten-1 bei üblichem Verlauf der Reaktion bevorzugt in 2,3-Dimethyl-1,2-butandiol hätte überführt werden müssen. Zwar ist bekannt, daß die Hydroxylierungsreaktion mit Wasserstoffperoxid in Gegenwart von Carbonsäure auch zu Umlagerungen führen kann. Jedoch werden stets nur Anteile des eingesetzten Olefins, nie aber das gesamte Olefin mit dem Ergebnis, daß ein einheitliches Reaktionsprodukt entsteht, umgelagert.

2,3-Dimethylbuten-1 und 2,3-Dimethylbuten-2 sind leicht zugängliche Einsatzstoffe. Sie werden z. B. durch Dimerisieren von Propylen erhalten und liegen dann in ausreichender Reinheit vor, um entweder nach vorheriger Destillation oder unmittelbar in den erfindungsgemäßen Prozeß eingesetzt zu werden.

Gemische aus 2,3-Dimethylbuten-1 und 2,3-Dimethylbuten-2 sind auch durch Dehydratisierung aus 2,3-Dimethylbutanol-1 in Gegenwart üblicher Dehydratisierungskatalysatoren wie Aluminiumoxid zugänglich. Sie können ebenfalls ohne vorherige Reinigungsschritte nach dem erfindungsgemäßen Verfahren umgesetzt werden.

Ein wesentliches Merkmal des neuen Verfahrens ist die getrennte Zuführung der Reaktanten Olefin und Wasserstoffperoxid in die vorgelegte Ameisensäure. Bei der technischen Durchführung der Umsetzung verfährt man in der Weise, daß man die Ameisensäure auf die Reaktionstemperatur, d. h. auf 50 bis 70 °C erhitzt und darauf Olefin und Wasserstoffperoxid in dem Maße zuführt, daß die frei werdende Reaktionsenthalpie ausreicht, die Reaktionstemperatur aufrechtzuerhalten. Das Wasserstoffperoxid wird in Form einer 30- bis 50gew.-%igen wäßrigen Lösung verwendet.

Die Ameisensäure liegt in reiner Form oder als Mischung mit bis zu 20 Gew.-% Wasser vor. Je Mol Olefin werden mindestens zwei Mol Ameisensäure eingesetzt, überschüssige Ameisensäure schadet nicht. Das Molverhältnis von Olefin und Wasserstoffperoxid beträgt 1 : 1.

Um eine innige Vermischung von Olefin und Wasserstoffperoxid zu gewährleisten, ist es erforderlich, das Reaktionsgemisch intensiv zu rühren.

Unter den Reaktionsbedingungen und der speziellen Art der Reaktionsführung setzt sich das Olefin spontan zum Monoformiat des Pinakols um. Nach Beendigung der Zugabe von Olefin und Wasserstoffperoxid läßt man bei der Reaktionstemperatur noch etwa eine Stunde nachreagieren und setzt darauf das Diol durch Verseifen mittels wäßrigem Alkalihydroxid frei.

Anschließend wird die organische Phase abgetrennt und die wäßrige Phase mit einem organischen Lösungsmittel, z. B. Isobutanol, extrahiert. Die vereinigten organischen Phasen werden durch fraktionierte Destillation zu reinem 2,3-Dimethyl-2,3-butandiol aufgearbeitet.

In den folgenden zwei Beispielen wird die Überlegenheit der erfindungsgemäßen Arbeitsweise (Ausführungsbeispiel) durch Vergleich mit einem Verfahren nach dem Stand der Technik (Vergleichsbeispiel) verdeutlicht.

Aus dem Ausführungsbeispiel wird das Ergebnis des erfindungsgemäßen Verfahrens ersichtlich.

Vergleichsbeispiel

In einem Rundkolben werden 325 g 85 %ige Ameisensäure und 293 g 36 %iges Wasserstoffperoxid auf 60 °C erwärmt. Über eine Pumpe werden anschließend 252 g 2,3-Dimethylbuten, bestehend aus 79 % 2,3-Dimethyl-2-buten und 21 % 2,3-Dimethyl-1-buten, in einem Zeitraum von einer Stunde zudosiert. Die dabei einsetzende Reaktion erfordert eine mäßige Kühlung. Nach weiteren zwei Stunden bei 60 °C ist das Reaktionsgemisch nahezu homogen. Zwecks Aufarbeitung wird nun mit Natronlauge neutralisiert, eine Phasentrennung durchgeführt und destilliert. Das Rohprodukt weist neben 43 % Pinakol und 38 % an nicht umgesetztem Einsatzolefin ca. 10 % 2,3-Dimethyl-1,2-butandiol auf. Infolge der eng beieinanderliegenden Siedepunkte von Pinakol und 2,3-Dimethyl-1,2-butandiol ist eine destillative Reindarstellung praktisch nicht realisierbar. Nach der Abtrennung des nicht umgesetzten Olefins, das erneut der Synthese zugeführt werden kann, wird als zweite Fraktion ein nur 90 %iges Pinakol als Kopfprodukt erhalten.

Ausführungsbeispiel

In einem Rundkolben werden 325 g 85 %ige Ameisensäure auf 50 °C erwärmt und unter Rühren über zwei separate Pumpen mit 293 g 36 %igem Wasserstoffperoxid und 252 g 2,3-Dimethylbuten (79 % 2,3-Dimethyl-2-buten, 21 % 2,3-Dimethyl-1-buten) mit solcher Geschwindigkeit versetzt, daß die Innentemperatur auf 60 °C gehalten wird. Zwei Stunden nach Beendigung der Zugabe ist die Reaktion abgeschlossen. Das vollständig homogene Reaktionsgemisch wird nun neutralisiert und von der Wasserphase befreit. Das Rohprodukt zeigt nach gaschromatographischer Analyse folgende Zusammensetzung : 80 % Pinakol, 7 % 2,3-Dimethylbuten und 0,1 % isomere Diole ; der Rest verteilt sich auf Aceton, Pinakolon und eine nicht näher identifizierte Vorlaufkomponente. Das Pinakol wurde durch seinen Siedepunkt von 105 °C bei 50 Torr und seinen Festpunkt von 42 bis 43 °C identifiziert.

Durch fraktionierte Destillation in Gegenwart eines geeigneten Wasserschleppmittels werden 241 g eines wasserfreien 99,6 %igen Pinakols erhalten.

Eine weitere allgemein anwendbare Methode zur Optimierung der Ausbeute besteht darin, daß man die wäßrige Phase mit einem geeigneten organischen Lösungsmittel (z. B. Isobutanol) extrahiert und die vereinigten Extrakte mit der den Hauptanteil Pinakol enthaltenden organischen Phase vereint und gemeinsam der Destillation unterwirft. Hierbei dient das Lösungsmittel gleichzeitig als Azeotropbildner zur Gewinnung von wasserfreiem Pinakol.

## Patentansprüche

1. Verfahren zur Herstellung von 2,3-Dimethyl-2,3-butandiol durch Umsetzung von 2,3-Dimethylbuten mit Wasserstoffperoxid und Ameisensäure und Spaltung des gebildeten Formiates, dadurch gekennzeichnet, daß man gleichzeitig aber getrennt ein Gemisch aus 2,3-Dimethylbuten mit bis zu 30 Gew.-% 2,3-Dimethylbuten-1 und Wasserstoffperoxid bei 50 bis 70 °C unter Rühren in Ameisensäure einleitet.

2. Verfahren nach Anspruch 1, dadurch gekennzeichnet, daß das Wasserstoffperoxid in Form einer 30- bis 50 %-igen, wässrigen Lösung eingesetzt wird.

3. Verfahren nach Anspruch 1 und 2, dadurch gekennzeichnet, daß die Ameisensäure in reiner Form oder als Mischung mit bis zu 20 Gew.-% Wasser eingesetzt wird.

## Claims

1. Process for the preparation of 2.3-dimethyl-2.3-butanediol by the reaction of 2.3-dimethylbutene with hydrogen pyroxide and formic acid and splitting of the formate thus formed, characterised in that a mixture of 2.3-dimethylbutene with up to 30 wt.% 2.3 dimethylbutene-1 and hydrogen peroxide is introduced simultaneously but separately at 50 to 70 °C into formic acid with stirring.

2. Process according to claim 1, characterised in that the hydrogen peroxide is added in the form of a 30 to 50 % aqueous solution.

3. Process according to claims 1 and 2, characterised in that the formic acid is added in pure form or as a mixture with up to 20 wt.% water.

## Revendications

1. Procédé pour la fabrication de 2,3-diméthyl-2,3-butanediol par réaction du 2,3-diméthylbutène avec le peroxyde d'hydrogène et l'acide formique et dissociation du formiate ainsi formé, caractérisé en ce que l'on introduit simultanément, mais séparément, un mélange de 2,3-diméthylbutène contenant jusqu'à 30 % en poids de 2,3-diméthylbutène-1 et de peroxyde d'hydrogène à 50-70 °C en agitant dans l'acide formique.

2. Procédé selon la revendication 1, caractérisé en ce que le peroxyde d'hydrogène est utilisé sous forme d'une solution aqueuse à 30-50 %.

3. Procédé selon la revendication 1 ou 2, caractérisé en ce que l'acide formique est utilisé sous forme pure ou en mélange avec jusqu'à 20 % en poids d'eau.